# FASCICULE DE BREVET EUROPEEN

(11) **EP 0 647 456 B1**
(45) Date de publication et mention de la délivrance du brevet: **03.07.2002**
(21) Numéro de dépôt: 94402222.7
(22) Date de dépôt: 05.10.1994
(51) Int. Cl.: A61N 1/39

(54) **Défibrillateur implantable à générateur de chocs isolé optiquement**
Implantierbarer Defibrillator mit einem optisch isolierten Elektroschockgenerator
Implantable defibrillator with an optically insulated electroshock generator

(30) Priorité: 08.10.1993 FR 9311993
(43) Date de publication de la demande: 12.04.1995
(73) Titulaire: ELA MEDICAL, F-92541 Montrouge (FR)
(72) Inventeur: Kroiss, Daniel, F-67590 Schweighouse-Moder (FR); Ostroff, Alan, F-67250 Preuschdorf (FR); Jacobson, Peter, F-67500 Heguenau (FR)
(74) Mandataire: Laget, Jean-Loup

(56) Documents cités:
- EP-A- 0 362 093
- EP-A- 0 473 002
- EP-A- 0 536 873
- EP-A- 0 553 864
- US-A- 4 800 883
- US-A- 4 823 796

## Description

L'invention concerne un circuit pour fournir des chocs à haute énergie dans un défibrillateur implantable.

Dans le cas présent, le terme défibrillateur se réfère à tout dispositif pour éliminer une tachyarythmie, avec une énergie électrique qui dépasse sensiblement l'énergie fournie par des stimulateurs cardiaques implantables, y compris toutes combinaisons ou sous-combinaisons de défibrillateurs, appareils de cardioversion et stimulateurs cardiaques implantables.

L'invention concerne plus particulièrement la commande de commutateurs électroniques isolés électriquement dans un défibrillateur implantable. L'invention peut être appliquée à l'envoi de chocs tronqués multiphasiques ou monophasiques vers une charge à partir d'un condensateur unique. Dans la description qui suit :
- Un choc monophasique envoie le courant dans un sens.
- Un choc multiphasique envoie le courant d'abord dans un sens (c'est la première phase du choc) puis dans le sens opposé (c'est la seconde phase), et peut fournir des phases alternées additionnelles. Un choc biphasique comprend deux phases.
- Un choc tronqué met brusquement fin à l'envoi de courant dans la charge, soit en interrompant le courant dans la charge soit en déchargeant rapidement le condensateur de stockage.
- La charge représente l'impédance des fils conducteurs, des électrodes de défibrillation, de l'interface électrodes-tissus, et de la masse des tissus entre les électrodes.
- Le condensateur unique représente toute combinaison série et/ou parallèle de groupes de condensateurs qui correspond à un unique condensateur équivalent dont les deux bornes sont reliées au circuit d'application de chocs.

L'invention concerne les générateurs de chocs pour formes d'onde monophasiques et multiphasiques, y compris des formes d'onde biphasiques. Elle s'applique à des générateurs à formes d'ondes multiphasiques à condensateur unique et à formes d'ondes multiphasiques à condensateurs multiples. La forme d'onde à condensateur unique décharge le condensateur dans la charge dans un premier sens pendant la première phase, interrompt le courant, continue de décharger le condensateur dans la charge dans le sens opposé pendant la seconde phase, puis tronque le choc. En conséquence, l'amplitude du front -avant de la seconde phase est égale à l'amplitude du front arrière de la première phase. La forme d'onde d'un condensateur multiple décharge un condensateur différent pour chaque sens, ou même pour chaque phase, et l'amplitude du front avant ne dépend pas nécessairement de l'amplitude du front arrière de la phase précédente.

Les anciens défibrillateurs ne fournissent que des formes d'ondes monophasiques. Le brevet USRE 27652 au nom de Mirowski (priorité du 9 fév. 1970) décrit un défibrillateur automatique à circuit d'application de chocs monophasiques qui fournit un choc non tronqué dès que le condensateur de stockage est chargé jusqu'à une tension fixe (aucun signal de commande isolé n'est nécessaire). Le brevet EP 0 362 093 décrit un défibrillateur implantable à un seul condensateur. Un éclateur est monté entre le condensateur et le coeur. Lorsque la tension de charge du condensateur atteint la tension de seuil de l'éclateur, celui-ci devient conducteur et décharge automatiquement et complètement le condensateur à travers le coeur. L'éclair d'amorçage de l'éclateur est capté par un récepteur photoélectrique qui inhibe le circuit de charge du condensateur. Le document US A 4 823 796 décrit un défibrillateur pour appliquer une impulsion trapézoïdale extérieure à un patient souffrant de fibrillation ventriculaire. Un condensateur de stockage d'énergie est relié aux électrodes sous le contrôle de moyens électroniques comportant une isolation optique. Le brevet FR 2 257 312 au nom de Zacouto (priorité du 16 janv. 1974) montre des moyens pour appliquer des chocs monophasiques séquentiels par des paires multiples d'électrodes, n'isolant pas non plus la commande.

Les brevets US 4 403 614 au nom de Engle (priorité du 19 Juillet 1979) et US 4 384 585 au nom de Zipes (priorité du 6 Mars 1981) décrivent des moyens pour synchroniser un choc avec des événements détectés, mais ne montrent aucun détail du circuit de décharge. Le brevet US 4 614 192 au nom de Imran (priorité du 21 Avril 1982) ajoute des moyens pour tronquer des chocs monophasiques en déchargeant rapidement le condensateur de stockage.

Le commutateur et la commande de chocs consistent en un transformateur d'impulsions commandant un thyristor.

Suite à des expériences concernant des chocs bidirectionnels en 1964 et 1980, J.C. Schuder et al ont décrit un " Ultrahigh-energy hydrogen thyratron / SCR bidirectional waveform defibrillator", dans Med Biol Eng Comput 20:419, 1982, consistant en un générateur biphasique avec un condensateur par phase. Le document SU 1149979 au nom de Pekarski (priorité du 8 oct. 1983) montre également un circuit d'application de chocs tronqués biphasiques avec un condensateur pour chaque phase.

En 1984, Schuder et al ont présenté les résultats d'une forme d'onde biphasique tronquée simulée à à condensateur unique. Dans leur rapport intitulé "Transthoracic Defibrillation of 100 Kg Calves with Bidirectional Truncated Exponential Shocks", vol. XXX Trans Am Soc Artif Intern Organs, 1984, les auteurs ont décrit des expériences faites avec une "forme d'onde asymétrique tronquée exponentielle biphasique... pouvant être mise en oeuvre dans un appareil de dimensions cliniques". Ils ont montré une forme d'onde où le front arrière de la première phase était égal au front avant de la seconde phase.

L'approche à condensateur unique simplifie à la fois la charge et la décharge des circuits, réduisant les dimensions, le poids et le risque de pannes des dispositifs implantables. A mesure que les données se sont accumulées, et ont montré les résultats améliorés obtenus sur des animaux et en clinique avec des chocs biphasiques tronqués par comparaison avec des chocs monophasiques tronqués , les concepteurs ont proposé une variété de générateurs à condensateur unique de formes d'onde multiphasiques tronquées.Tous ces circuits comprennent au moins quatre commutateurs dans une configuration de pont en H.

Les concepteurs utilisent fréquemment la configuration de pont en H pour envoyer du courant dans une charge dans deux directions à partir d'une source de courant continu, par exemple, pour commander un moteur pas à pas ou un servomoteur à partir d'une pile. Pendant la première phase, un premier commutateur relie le pôle positif de la source à un premier côté de la charge et un deuxième commutateur relie le pôle négatif de la source au second côté de la charge. Pendant la seconde phase, un troisième commutateur relie le pôle positif de la source au second côté de la charge, et un quatrième commutateur relie le pôle négatif de la source au premier côté de la charge. Les premier et troisième commutateurs, reliés au pôle positif de la source, sont appelés commutateurs du côté supérieur. Les second et quatrième commutateurs, qui sont reliés au pôle négatif de la source, sont appelés commutateurs du côté inférieur.

Les circuits à décharge implantables de l'art antérieur utilisent un ou plusieurs de trois types de commutateurs dans le pont en H. Chaque type de commutateur comprend une borne d'entrée, une borne de sortie et une borne de commande, et répond à un signal de commande appliqué entre les bornes de commande et de sortie. Les thyristors (SCR) deviennent conducteurs en réponse à une impulsion appliquée à la borne de commande, et ne cessent de fonctionner que lorsque le courant qui les traverse tombe sensiblement à zéro. Les transistors à effet de champ à métal-oxyde-semiconducteur (MOSFET) et les transistors bipolaires à gachette isolée (IGBT) restent passants tant qu'une tension de commande est appliquée à la borne de commande.

Selon la façon dont ils protègent les circuits de stimulation et de détection vis-à-vis d'impulsions de défibrillation, les circuits de l'art antérieur soit isolent le condensateur et le circuit de décharge de la masse de stimulation et de détection, soit relient le côté négatif du condensateur à la masse. Dans la version isolée, ils doivent fournir des signaux isolés de commande des commutateurs. Dans la version à masse négative, ils doivent toujours appliquer des signaux de commande isolés aux commutateurs du côté supérieur.

Ainsi, tout circuit d'application de chocs biphasiques à condensateur unique a besoin de deux commutateurs du côté supérieur et deux commutateurs du côté inférieur reliés en un pont en H, et au moins deux commandes isolées de commutateurs. Les brevets de l'art antérieur qui suivent décrivent tous un pont en H pour générer une forme d'onde multiphasique à condensateur unique, où la structure des moyens de commutation et des moyens de commande des commutateurs diffère dans chaque conception:

Le brevet US 4 800 883 au nom de Winstrom (priorité du 2 avril 1986) propose un circuit de décharge isolé avec quatre commutateurs MOSFET, et utilise un transformateur alimenté en radiofréquence, un redressement, et des circuits de coupure rapide pour les commandes du côté supérieur et du côté inférieur. Un unique transformateur avec deux secondaires commande à la fois les commutateurs du côté supérieur et du côté inférieur dans la même phase. Toutes ses revendications se rapportent à un condensateur à niveaux multiples avec des prises de tension.

Le brevet EP 0281219 au nom de Mehra (priorité du 14 janvier 1987) propose un circuit de décharge à masse négative avec un SCR en série avec un MOSFET pour chaque commutateur du côté supérieur et un SCR pour chaque commutateur du côté inférieur. Mehra ne donne pas de détails sur les commandes des commutateurs.

Le brevet EP 0280526 au nom de Baker (priorité du 27 fév. 1987) utilise le circuit de Winstrom ci-dessus, avec la condition supplémentaire d'une durée de la première phase plus longue que la durée de la seconde phase (à noter qu'en 1984, Jones et al ont publié des résultats pour des impulsions de défibrillation avec une première phase de 5 ms et une seconde phase de 1 ms, voir Am. J. Physiol. 247 (Heart Circ. Physiol. 16). Le document EP 0324380 au nom de Bach (priorité du 12 janv. 1988) propose un autre circuit de décharge à masse négative avec des SCR pour les commutateurs du côté supérieur et des MOSFET pour les commutateurs du côté inférieur. Bach utilise des transformateurs d'impulsions pour les commandes du côté supérieur et commande directement le côté inférieur. Le brevet EP 0326290 au nom de De Coriolis (priorité du 19 janv. 1988) propose un autre circuit de décharge à masse négative avec deux SCR en série pour le commutateur du côté supérieur de la première phase, un MOSFET pour le commutateur du côté inférieur de la première phase, et des SCR pour les commutateurs du côté supérieur et du côté inférieur de la seconde phase. De Coriolis tronque la seconde phase par une décharge rapide du condensateur de stockage par le commutateur du côté supérieur de la première phase et le commutateur du côté inférieur de la seconde phase. De Coriolis commande les commutateurs du côté supérieur avec des transformateurs d'impulsions et les commutateurs du côté inférieur avec des décaleurs de niveau raccordés à une alimentation positive.

Le brevet US 4 998 531 au nom de Bocchi (priorité du 28 mars 1990) propose un autre circuit de décharge à masse négative avec quatre commutateurs MOSFET. Bocchi utilise des décaleurs de niveau pour les commandes du côté inférieur et utilise un transformateur pour la commande du côté supérieur, où une impulsion dans un sens rend passant le MOSFET et une impulsion dans le sens inverse le bloque.

Le brevet US 5 111 816 au nom de Pless (priorité du 22 oct. 1990) propose un autre circuit de décharge à masse négative avec des commutateurs IGBT et MOSFET. Toutes les variantes de Pless commandent à la fois les commutateurs du côté supérieur et du côté inférieur dans la même phase à partir d'un transformateur commun alimenté en radiofréquence (RF) et un redressement, et un circuit de coupure rapide pour au moins un commutateur de chaque phase. Pless raccorde également la borne négative de la pile à la masse et l'inverse pour établir la tension de stimulation.

Il y a cependant possibilité d'améliorer les circuits d'application de chocs à haute tension dans des défibrillateurs implantables.

Toutes les conceptions de l'art antérieur soit isolent le circuit de décharge de la masse de stimulation et de détection, soit relient le pôle négatif du condensateur de stockage à la masse de stimulation et de détection. Ceci exige d'isoler électriquement les signaux de commande pour les commutateurs du côté supérieur. Toutes les conceptions de l'art antérieur utilisent un couplage par transformateur pour cette isolation. Elles utilisent des transformateurs d'impulsion pour commander des SCR, et soit des transformateurs d'impulsion, soit des transformateurs en radiofréquence (RF) avec redressement et circuit de coupure rapide, pour commander les MOSFET ou les IGBT.

Les inconvénients du couplage par transformateur comprennent:
- le couplage magnétique avec d'autres inducteurs ou transformateurs dans l'implant, tels que le transformateur qui charge les condensateurs de stockage d'énergie,
- le couplage magnétique avec des circuits sensibles situés ailleurs dans l'implant, tels que des boucles de courant dans les circuits de détection d'onde R à gain élevé,
- des composants magnétiques relativement volumineux et coûteux qui ne peuvent pas être mis en oeuvre en technologie à circuits intégrés,
- la possibilité d'une saturation du noyau du transformateur dans un champ magnétique élevé continu, y compris les champs produits par des aimants permanents utilisés habituellement pour tester les stimulateurs cardiaques et les défibrillateurs,
- le couplage magnétique avec de forts champs magnétiques dus à un courant alternatif externe tels que les champs produits par les appareils de chauffage ou de soudage industriels.

Dans les conceptions de l'art antérieur, les transformateurs exigent également un oscillateur à radiofréquence additionnel complexe et consommateur d'énergie, ou des circuits de commande d'impulsion.

Le but principal de l'invention est donc d'éviter ces inconvénients dans des circuits de commande pour commutateurs d'application de chocs à haute tension en fournissant une commande optiquement isolée pour commutateurs électroniques isolés électriquement . La commande optique a besoin d'un émetteur pour convertir un signal électrique en un signal optique, d'un trajet optique isolé électriquement, et d'un récepteur pour convertir le signal optique en un signal électrique.

Une variante de l'invention fournit un trajet de communication optique pour le signal de commande pour chaque commutateur, où la présence du signal optique informe le récepteur qu'il doit fermer le commutateur commandé, alors que son absence indique au récepteur qu'il doit l'ouvrir . Une autre variante de l'invention propose deux trajets de communication optique pour commander chaque commutateur isolé, où la présence d'un signal dans le premier trajet indique au récepteur qu'il doit fermer le commutateur commandé, et la présence d'un second signal optique dans le second trajet indique au récepteur qu'il doit ouvrir le commutateur commandé.

Un autre but encore de l'invention est de proposer des circuits récepteurs optiques pour la commande isolée des commutateurs. Une variante du circuit récepteur prévoit une alimentation flottante et un phototransistor pour appliquer le courant de charge aux bornes de commande du commutateur isolé commandé en réponse à un signal de commande optique. Une autre variante propose une ou plusieurs photodiodes pour remplir la même fonction.

Un autre but encore de l'invention est de proposer des circuits pour ouvrir rapidement le commutateur commandé en réponse à un état particulier du ou des signaux de commande optiques.

L'invention a pour objet un défibrillateur implantable à générateur de chocs comprenant :
- une pile pour fournir l'énergie,
- des circuits de commande pour déterminer les instants de choc,
- un circuit de charge de choc pour convertir l'énergie de la pile en énergie de choc,
- un condensateur pour stocker l'énergie de choc,
- des moyens de commutation électroniques pour relier ledit condensateur à une charge, et
   . des moyens comportant une isolation optique pour commander les moyens de commutation électroniques, et comprenant:
      . des moyens d'émission optiques pour convertir au moins un signal électrique provenant desdits circuits de commande en au moins un signal optique,
      . des moyens de réception optiques pour convertir au moins un signal optique en au moins un signal électrique pour fermer et ouvrir selectivement ledit commutateur électronique, et
      . un trajet optique isolé électriquement acheminant chaque signal optique provenant desdits moyens d'émission optiques vers lesdits moyens de réception optiques,
- lesdits moyens d'émission optiques émettant un signal optique dont la présence indique auxdits moyens de réception de fermer ledit commutateur électronique, et dont l'absence indique auxdits moyens de réception d'ouvrir ledit commutateur électronique,
- lesdits moyens de réception comprenant :
   . des moyens d'alimentation pour fournir la puissance qui charge les bornes de commande dudit commutateur électronique,
   . un premier commutateur à phototransistor pour diriger sélectivement le courant provenant desdits moyens d'alimentation vers les bornes de commande dudit commutateur électronique, pour charger les bornes de commande dudit commutateur électronique et ainsi fermer ledit commutateur électronique en réponse à la présence dudit signal optique,
caractérisé en ce que lesdits moyens de réception comprennent :
- des moyens de coupure pour décharger sélectivement les bornes de commande dudit commutateur électronique et ainsi ouvrir ledit commutateur électronique en réponse à l'absence dudit courant de charge.

Lesdits moyens d'alimentation comprennent :
- un condensateur pour stocker de l'énergie à basse tension,
- un circuit à courant limité, comprenant au moins une résistance de valeur élevée, pour charger ledit condensateur pour stocker l'énergie à basse tension en provenance dudit condensateur pour stocker l'énergie de choc, et
- des moyens limiteurs de tension pour empêcher ledit condensateur de stockage d'énergie à basse tension de se charger au-delà d'une tension préétablie.

Lesdits moyens limiteurs de tension comprennent, une diode de Zener avec une tension de Zener d'approximativement 15 V.

Lesdits moyens de coupure comprennent des moyens à transistor pour décharger les bornes de commande dudit commutateur électronique quand lesdits moyens d'alimentation et lesdits moyens à phototransistor ne fournissent plus de courant.

Lesdits moyens de réception optiques comprennent :
- au moins une photodiode pour fournir sélectivement le courant aux bornes de commande dudit commutateur électronique pour charger les bornes de commande dudit commutateur électronique et fermer ainsi ledit commutateur électronique en réponse à la présence dudit signal optique, et
- des moyens de coupure pour décharger sélectivement les bornes de commande dudit commutateur électronique et ainsi ouvrir ledit commutateur électronique, en réponse à l'absence dudit courant de charge en provenance de ladite ou lesdites photodiodes.

Lesdits moyens de coupure comprennent des moyens à transistor pour décharger les bornes de commande dudit commutateur électronique quand ladite ou lesdites photodiodes ne fournissent plus de courant aux bornes de commande dudit commutateur électronique.

Lesdits moyens d'émission optiques émettent un premier signal optique dont la présence indique auxdits moyens de réception de fermer ledit commutateur électronique, et émettent un second signal optique dont la présence indique auxdits moyens de réception d'ouvrir ledit commutateur électronique.

Lesdits moyens de réception comprennent :
- des moyens d'alimentation pour fournir la puissance qui charge les bornes de commande dudit commutateur électronique,
- un premier commutateur à phototransistor pour faire passer sélectivement la puissance depuis lesdits moyens d'alimentation vers les bornes de commande dudit commutateur électronique, pour charger les bornes de commande dudit commutateur électronique et ainsi fermer ledit commutateur électronique, en réponse à la présence dudit premier signal optique, et
- des moyens de coupure pour décharger sélectivement les bornes de commande dudit commutateur électronique et ainsi ouvrir ledit commutateur électronique en réponse à la présence dudit second signal optique.

Lesdits moyens de coupure comprennent :
- un second phototransistor activé par ledit second signal optique, relié de façon à décharger les bornes de commande dudit commutateur électronique quand il est activé, et
- une résistance pour empêcher l'accumulation de la charge auxdites bornes de commande quand aucun signal optique n'est présent.

Lesdits moyens de réception optiques comprennent :
- au moins une photodiode pour fournir sélectivement le courant aux bornes de commande dudit commutateur électronique pour charger les bornes de commande dudit commutateur électronique et ainsi fermer ledit commutateur électronique, en réponse à la présence dudit premier signal optique, et
- des moyens de coupure pour décharger sélectivement les bornes de commande dudit commutateur électronique et ainsi ouvrir ledit commutateur électronique, en réponse à la présence dudit second signal optique.

Lesdits moyens de coupure comprennent :
- un second phototransistor activé par ledit second signal optique, relié de façon à décharger les bornes de commande dudit commutateur électronique quand il est activé, et
- une résistance pour empêcher l'accumulation de la charge sur lesdites bornes de commande quand aucun signal optique n'est présent.

Lesdits moyens de commutation électroniques comprennent deux commutateurs électroniques sur le côté supérieur et deux commutateurs électroniques sur le côté inférieur, en configuration de pont en H pour relier ledit condensateur à une charge avec une polarité sélective.

Ledit défibrillateur comprend en outre une liaison entre le côté inférieur dudit condensateur et dudit pont en H et une tension d'alimentation à la masse.

Ledit défibrillateur comprend en outre une liaison entre le côté inférieur dudit condensateur et dudit pont en H et une tension d'alimentation plus négative que la masse.

Ladite tension d'alimentation est comprise entre -5 et -20 V.

Ladite tension d'alimentation est d'approximativement -15 V.

Lesdits moyens pour commander les moyens de commutation électroniques comprennent :
- deux commandes isolées du côté supérieur pour commander sélectivement chaque commutateur du côté supérieur en réponse à un signal correspondant provenant desdits circuits de commande, et
- deux commandes du côté inférieur pour commander sélectivement chaque moyen de commutation du côté inférieur en réponse à un signal correspondant provenant desdits circuits de commande,
les commandes du côté supérieur étant isolées optiquement.

Lesdits circuits de commande pour déterminer les durées de chocs activent d'abord l'une desdites commandes du côté supérieur, puis après une certaine durée préétablie activent la commande correspondante du côté inférieur, pour commencer chaque phase d'un choc.

Lesdits circuits de commande pour les temps de chocs commencent par désactiver l'une desdites commandes du côté supérieur, puis après une certaine durée préétablie, désactivent la commande correspondante du côté inférieur, pour mettre fin à chaque phase d'un choc.

Lesdits circuits de commande pour déterminer les durées de chocs désactivent simultanément l'une desdites commandes du côté supérieur et la commande correspondante du côté inférieur pour mettre fin à chaque phase d'un choc.

La durée préétablie est de l'ordre de quelques centaines de microsecondes.

L'invention est décrite ci-après avec référence aux dessins annexés dans lesquels :
La figure 1 est une vue d'ensemble de l'invention appliquée à un générateur de chocs multiphasiques d'un défibrillateur implantable.
La figure 2 montre l'émetteur, le récepteur et le trajet optique pour commander un unique commutateur isolé.
La figure 3 montre un émetteur pour une variante de l'invention avec un signal optique par commutateur isolé commandé.
La figure 4 montre un émetteur pour une autre variante de l'invention, avec deux signaux optiques pour chaque commutateur isolé commandé.
La figure 5 montre un récepteur pour une variante de l'invention utilisant une alimentation isolée et un phototransistor.
La figure 6 montre un récepteur pour l'autre variante de l'invention, utilisant des photodiodes.
La figure 7 montre un circuit de coupure utilisé avec la variante de l'invention comprenant deux trajets optiques pour chaque commutateur isolé commandé.
Les figures 8 à 12 montrent d'autres circuits de coupure utilisés avec la variante de l'invention comprenant un trajet optique pour chaque commutateur isolé commandé.
Les figures 13 et 14 montrent la séquence des signaux de commande pour appliquer le procédé de l'invention à un générateur de chocs multiphasiques.

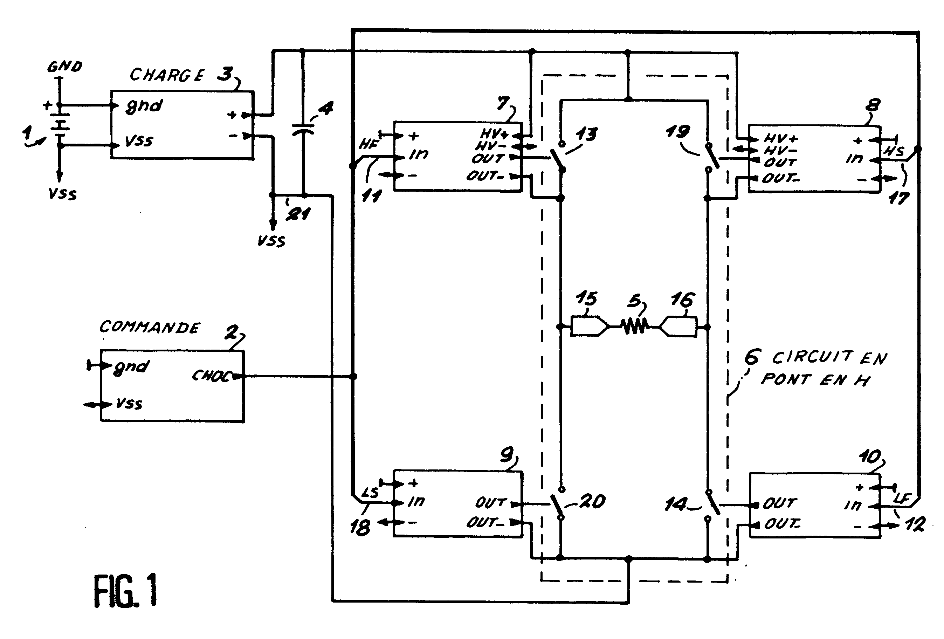

En se référant à la figure 1, une pile 1 fournit l'énergie aux circuits d'un défibrillateur implantable, typiquement de 5 à 15V. Les circuits de commande 2 qui déterminent la séquence des chocs fournissent des signaux de commande aux commutateurs électroniques du générateur de chocs, et dans cet exemple fournissent des chocs biphasiques à partir d'un condensateur unique. Un circuit de charge pour les chocs 3 convertit l'énergie de la pile en énergie de choc, typiquement de 0,75 kV, stockée dans un condensateur 4, qui est typiquement de 125 µF.

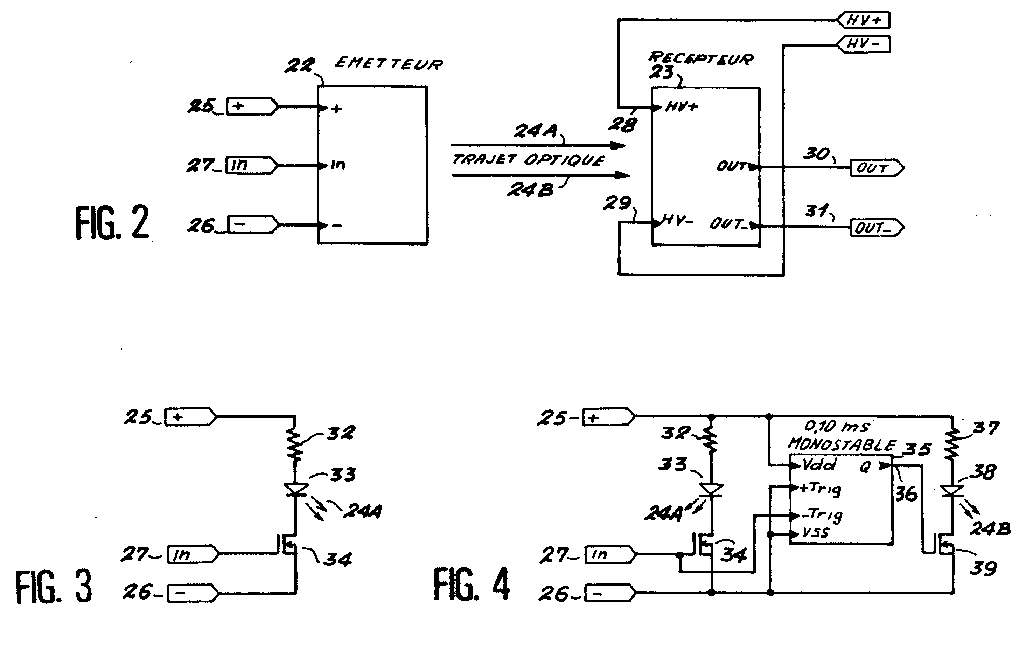

La figure 1 montre des moyens de commutation électroniques pour relier le condensateur 4 à une charge 5, les moyens de commutation consistant en un circuit à pont en H 6. Elle montre également des commandes 7 et 8 du côté supérieur ou des commandes 9 et 10 du côté inférieur, pour actionner les commutateurs du pont en H 6. Les commandes 7, 8, à isolation optique sont décrites en relation avec la figure 2.

A la figure 1, quand les circuits de commande 2 établissent les signaux de commande HF en 11 et LF en 12, la commande 7 du côté supérieur et la commande 10 du côté inférieur acheminent ces signaux de commande de façon qu'ils ferment les commutateurs 13 et 14 respectivement, de manière que le courant passe du condensateur 4 dans la charge 5 dans un premier sens, de 15 vers 16 (ceci constituant la première phase du choc). Ensuite, les circuits de commande inhibent toutes les sorties de commande 11, 12, 17, 18, ouvrant tous les commutateurs. Ceci détermine un délai entre les phases, ce qui donne le temps à tous les commutateurs de s'ouvrir. Ensuite, les circuits de commande établissent les signaux HS en 17 et LS en 18, ce qui fait que la commande 8 du côté supérieur et la commande 9 du côté inférieur ferment les commutateurs 19 et 20 respectivement, et que le courant passe du condensateur 4 dans la charge 5 dans un second sens, de 16 vers 15 (ceci constituant la seconde phase du choc). Ensuite, les circuits inhibent toutes les sorties de commande 11, 12, 17, 18, ouvrant tous les commutateurs et tronquant la seconde phase. Les circuits de commande peuvent optionnellement poursuivre cette séquence pour générer des phases additionnelles.

Les commutateurs 13, 14, 19 et 20 de la figure 1 peuvent être constitués par des MOSFET ou des IGBT, ainsi que cela est connu de l'homme de l'art. Les MOSFET et les IGBT doivent comprendre des diodes en série, comme montré dans l'art antérieur, pour éviter qu'une défibrillation externe soit conduite dans le sens inverse à travers les commutateurs. Les commutateurs doivent avoir des caractéristiques nominales d'approximativement 30 A et 1,0 kV, et leur fuite à l'état non passant ne doit pas dépasser quelques micro-ampères.

Les commandes du côté inférieur, 9 et 10 de la figure 1, peuvent être constituées d'une manière classique, du fait qu'elles ne sont pas isolées. En général, des MOSFET qui ont une résistance de quelques centaines d'ohms sont utilisés dans une configuration push-pull. Ceci donne un temps de montée et un temps de descente de la tension de commande pour des transistors de commutation typiques 14, 20, d'approximativement 10 µs. Un condensateur de quelques nanofarads (non représenté) peut être éventuellement ajouté entre les bornes de sortie de la commande du commutateur pour contrôler le temps de montée et réduire l'importance de l'effet Miller. Il est important de maintenir un front de montée modéré pour l'impulsion de choc (de l'ordre de quelques ampères par microseconde) pour réduire le couplage inductif ou capacitif de l'impulsion de choc vers d'autres circuits sensibles du défibrillateur implantable, tels que des circuits de télémétrie et des amplificateurs de détection d'onde P ou d'onde R.

La figure 1 montre en outre une liaison 21 entre le côté inférieur du condensateur 4 et le côté inférieur du pont en H 6, et une alimentation négative VSS proche de la masse GND. Ceci permet de faire fonctionner les circuits de commande en 2 et les commandes 9 et 10 du côté inférieur entre la masse et cette tension d'alimentation négative, ce qui simplifie leurs circuits. Cependant, l'invention pourrait être également appliquée à des générateurs de chocs qui raccordent le côté inférieur à la masse, ou qui isolent le côté inférieur.

Les commandes 7, 8, 9, 10 comportent une entrée "in", une sortie "out" et une sortie négative "out -". Les références "HV +" et "HV -" représentent la haute tension positive et négative, respectivement. Ces références se trouvent aussi sur d'autres figures.

De nombreuses variantes de la figure 1 existent dans les limites du champ d'application de l'invention. Par exemple, un circuit d'alimentation additionnel pourrait être utilisé pour fournir des alimentations négatives séparées pour faire fonctionner le circuit de commande 2, le circuit de stimulation et de détection non représenté, et les commandes 9 et 10 du côté inférieur, au lieu de faire fonctionner ces circuits directement à partir de la pile. Des circuits à décalage de niveau pourraient être utilisés pour faire passer des signaux logiques référencés à une alimentation, à une autre référence. Ce circuit additionnel n'est pas représenté ici pour des raisons de clarté.

La figure 2 représente un exemple de réalisation d'une commande telle que 7 ou 8 de la figure 1.

La figure 2 montre l'émetteur 22, le récepteur 23 et un trajet optique 24 pour commander un commutateur isolé unique. L'émetteur 22 comporte des entrées d'alimentation "+" en 25 et "-" en 26, et une entrée de signal de commande "in" en 27. L'entrée 27 est un signal de niveau logique fonctionnant entre GND en 25 et VSS en 26. Le récepteur 23 comporte des entrées d'alimentation de puissance "HV+" en 28 et "HV-" en 29. Son signal de sortie "out" est en 30, et il est défini par référence à "out-" en 31. Une première variante de l'invention qui utilise un seul trajet optique 24A par commutateur commandé, et une seconde variante qui utilise deux trajets optiques 24A et 24B, sont décrites ci-après.

Quand le circuit de commande 2 de la figure 1 applique un signal "in" à l'état haut en 27, l'émetteur envoie alors un signal optique par le trajet 24A vers le récepteur, qui répond en appliquant un signal "out" à l'état haut en 30. Quand le circuit de commande met "in" à l'état bas, alors dans une première vanriante de l'invention, l'émetteur arrête d'envoyer le signal optique, et le récepteur répond en mettant "out" à l'état bas en 30. Dans une seconde variante, l'émetteur arrête l'envoi du premier signal optique et envoie une brève impulsion d'un second signal optique par le trajet 24B, que le récepteur utilise pour désactiver la sortie en 30.

La figure 3 montre un circuit émetteur pour la première variante de l'invention décrite ci-dessus, qui utilise un unique trajet optique. Quand le circuit de commande en 2 de la figure 1 établit "in" en 27 à un niveau haut, ceci rend passant le MOSFET à canal N en 34, permettant au courant de passer par la résistance de limitation 32 et la diode émettrice de lumière (LED) 33, jusqu'à ce que le circuit de commande ramène "in" au niveau bas. Un courant typique de la LED est d'approximativement 30 à 100 mA. Pendant que passe le courant, la diode envoie de la lumière le long du trajet optique 24A de la figure 2 pour acheminer l'information au récepteur 23 de la figure 2. La présence de ce signal optique indique au récepteur qu'il doit fermer le commutateur électronique, et son absence indique au récepteur qu'il doit ouvrir le commutateur électronique.

La figure 4 montre un circuit émetteur pour la seconde variante de l'invention décrite ci-dessus, utilisant deux trajets optiques. Les composants 25 à 27 et 32 à 34 fonctionnent comme décrit dans l'explication de la figure 3, sauf qu'il y a deux trajets optiques 24A, 24B. La LED 33 n'émet que par le trajet 24A. Ainsi, l'émetteur émet un premier signal optique dont la présence indique au récepteur qu'il doit fermer le commutateur électronique commandé. Quand le circuit de commande 2 fait passer "in" en 27 à un niveau bas, ceci éteint la LED en 33 et déclenche le multivibrateur monostable 35. Le monostable définit une période d'approximativement 0,10 ms pendant laquelle sa sortie Q en 36 reste à un niveau haut. Quand Q en 36 est à un niveau haut, ceci rend passant le MOSFET à canal N 39, en permettant au courant de passer par la résistance de limitation 37 et la LED 38, courant qui dans ce cas également est typiquement d'approximativement 30 à 100 mA. La LED 38 envoie de la lumière le long d'un second trajet optique 24B de la figure 2. La présence de ce second signal optique indique au récepteur 23 de la figure 2 qu'il doit ouvrir le commutateur commandé.

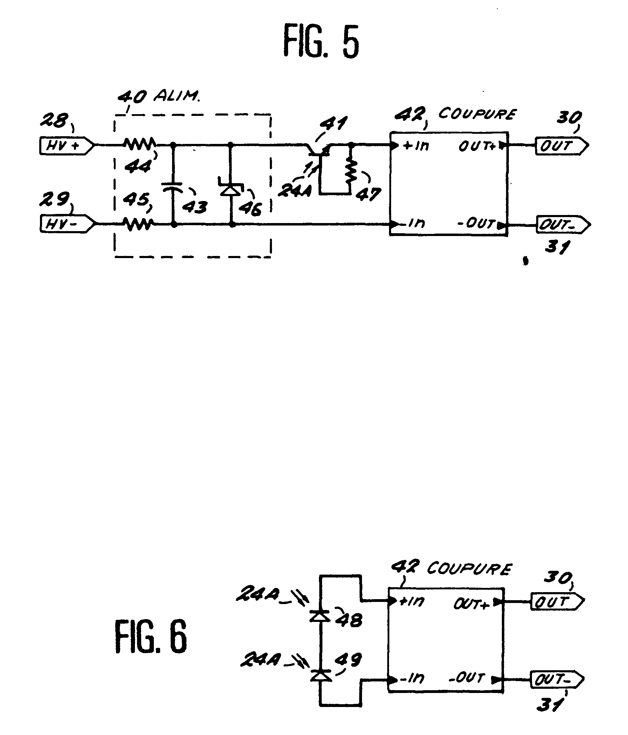

La figure 5 montre un circuit récepteur 23 (de la figure 2) consistant en : une alimentation Alim. 40 pour fournir la puissance qui charge les bornes de commande du commutateur commandé, un commutateur à phototransistor 41 pour conduire le courant de l'alimentation 40 vers les bornes de commande du commutateur commandé, quand il est activé par un signal optique selon le trajet optique 24A de la figure 2. La résistance 47, qui est typiquement de 0,47 Mégohms, aide à bloquer plus rapidement le phototransistor 41. La figure 5 montre également un circuit de coupure 42 pour décharger les bornes de commande du commutateur commandé quand le commutateur à transistor 41 cesse de fournir du courant, dans la première variante de l'invention, ou quand le circuit de coupure reçoit un second signal optique selon le trajet 24B (non montré), dans la seconde variante de l'invention.

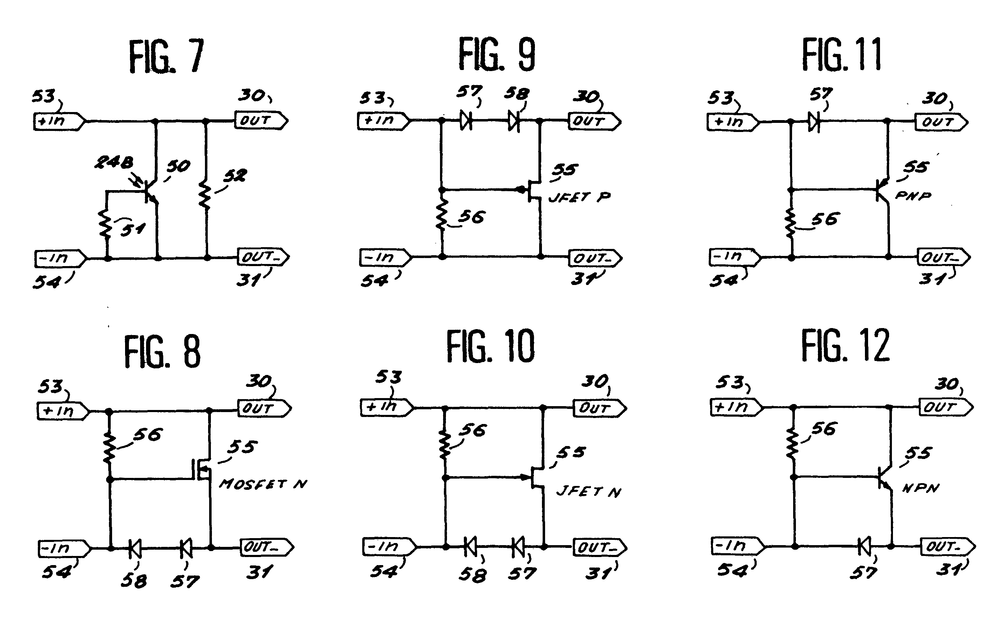

L'alimentation 40 de la figure 5 comprend un condensateur 43, typiquement de 50 nF, pour stocker l'énergie à basse tension, et un trajet à courant limité, avec des résistances de valeur élevée 44 et 45, pour relier le condensateur 43 à la charge à travers le condensateur d'alimentation à haute tension 4 du défibrillateur de la figure 1. Comme la valeur de la résistance est élevée, typiquement de 10 Mégohms, seul un faible courant passe dans la charge 5 de la figure 1 en raison des résistances 44 et 45. (Il est évident que lorsque l'alimentation 40 est utilisée en conjonction avec les circuits de décharge montrés aux figures 8, 10 ou 12, ce courant peut être réduit à une valeur très faible en raison des diodes de blocage 57, 58 montrées dans ces circuits). L'alimentation 40 de la figure 5 comprend également un limiteur de tension en 46 pour éviter une surcharge du condensateur 43. Le limiteur 46 peut être une diode de Zener avec une tension Zener d'approximativement 15 V.

La figure 6 montre un autre conception de récepteur utilisant au moins une photodiode (deux étant montrées en 48 et 49) pour remplacer l'alimentation en puissance 40 et le commutateur à phototransistor 41 de la figure 5, pour fournir un courant de charge aux bornes de commande du commutateur commandé en réponse au signal optique en 24A. Les photodiodes fournissent un courant qui n'est que de quelques pour cent du courant de la LED 33 de l'émetteur, à quelques volts par photodiode, sans aucune autre alimentation. Ceci simplifie considérablement le circuit et réduit le nombre des composants. Il est possible de placer des photodiodes additionnelles en série pour augmenter la tension de sortie, ou en parallèle pour augmenter le courant. En général, il est avantageux de prévoir au moins 0,15 mA à 15 V pour commander des commutateurs MOSFET ou IGBT typiques. La figure 6 comprend également un circuit de coupure 42 pour décharger les bornes de commande du commutateur commandé, comme expliqué dans la description de la figure 5.

Les figures 7 à 12 montrent différents circuits de coupure 42. Le circuit de la figure 7 est utilisé avec la seconde variante de l'invention décrite ci-dessus comprenant deux trajets optiques, et les autres circuits sont utilisés avec la première variante à trajet optique unique. Chacun de ces circuits est relié par ses entrées 53, 54 aux générateurs de courant montrés aux figures 5 ou 6. Le courant entre par la borne 53 et sort par la borne 54. Chacun de ces circuits est relié par des sorties 30 et 31 aux bornes de commande du commutateur commandé. Quand le circuit de coupure est activé, il décharge les bornes de commande en établissant un trajet de faible résistance entre 30 et 31.

Le circuit de la figure 7 est activé par un signal optique sur le trajet 24B comme décrit ci-dessus. Ceci rend passant le phototransistor 50 qui établit un trajet de faible résistance entre 30 et 31. La résistance 51, typiquement de 0,47 Mégohms, augmente l'immunité au bruit du phototransistor. La résistance 52, typiquement de 1,0 Mégohm empêche la montée de la charge sur les bornes de commande 30 et 31 qui pourrait déclencher de façon erronée un choc pendant la période entre chocs.

Les figures 8 à 12 montrent les circuits de coupure qui sont normalement activés, et qui sont désactivés par le courant qui entre par la borne 53 et qui sort par la borne 54. Ainsi, ces circuits maintiennent normalement le commutateur commandé (dont les bornes de commande sont en 30 et 31) à l'état ouvert, jusqu'à ce que l'alimentation et le phototransistor ou le circuit du photocoupleur des figures 5 et 6 envoie du courant pour fermer le commutateur commandé. Chaque circuit utilise un transistor 55 pour établir un trajet de faible impédance entre 30 et 31. Chaque circuit comprend une résistance de polarisation 56, typiquement de 1,5 Mégohms, pour maintenir le transistor 55 à l'état passant quand aucun courant ne passe entre les bornes d'entrée 53 et 54. Chaque circuit utilise une ou deux diodes à faible signal typiquement avec des caractéristiques de 0,10 A et 15 PIV, en 57 et 58, qui procurent une baisse de tension pour dépolariser le transistor quand le courant circule vers les bornes de commande 30, 31 du commutateur.
- La figure 8 montre le circuit de coupure mis en oeuvre avec un MOSFET à canal N du type à zone de dépletion, qui est passant avec une tension gâchette-source de zéro, et bloqué quand la tension de gâchette est plus négative de quelques volts que la source.
- La figure 9 utilise un transistor à effet de champ à jonction à canal P (JFET) qui est passant avec une tension gâchette-source de zéro, et bloqué quand la tension de gâchette est plus positive de quelques volts que la source.
- La figure 10 utilise un JFET à canal N, qui fonctionne comme le MOSFET à canal N du type à zone de dépletion de la figure 8.
- La figure 11 utilise un transistor pnp bipolaire qui est passant quand l'émetteur est plus positif d'environ 0,7 V que la base. Ainsi, dès que la borne de commande de commutateur 30 monte au-dessus de cette valeur par rapport à la borne 31, et qu'aucun courant ne passe par la diode 57, le transistor 55 est alors passant et maintient la tension de commande à environ 0,7 V au maximum. Quand le courant passe par la diode 57 pour fermer le commutateur commandé, ceci bloque le transistor 55.
- La figure 12 utilise un transistor npn bipolaire qui est passant quand la base est plus positive d'environ 0,7 V que l'émetteur. Le circuit fonctionne de façon similaire au circuit de la figure 11.

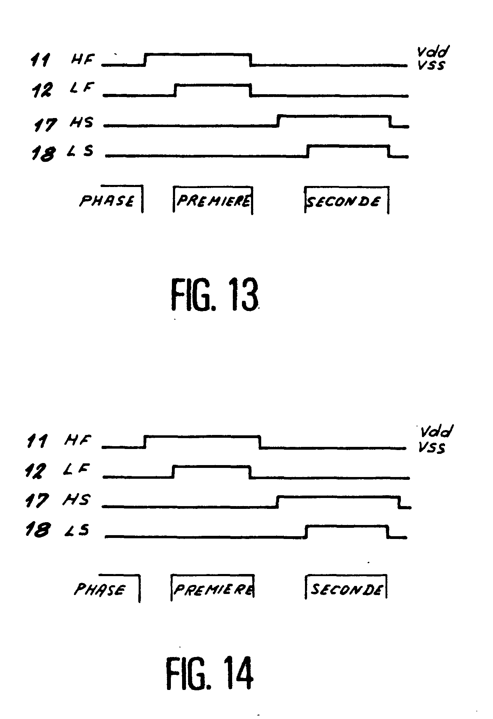

Les figures 13 et 14 montrent les séquences des quatre signaux de commande de commutateurs montrés à la figure 1. Chaque signal active une commande de commutateur qui, de son côté, actionne un commutateur comme montré sur le Tableau ci-dessous:

| **Nom des signaux** | **Numéros des signaux** | **Numéros des commandes** | **Numéros des commutateurs** |
|---|---|---|---|
| HF | 11 | 7 | 13 |
| | | | |
| LF | 12 | 10 | 14 |
| | | | |
| HS | 17 | 8 | 19 |
| | | | |
| LS | 18 | 9 | 20 |

Les commandes du côté supérieur utilisant un couplage optique, et notamment celles qui utilisent des photodiodes peuvent avoir besoin d'un temps assez long pour rendre passant ou bloquer le transistor commandé. Ceci peut être de l'ordre de quelques centaines de microsecondes, selon le rendement du transfert optique et le courant dans la LED de l'émetteur.

Quand un commutateur se ferme ou s'ouvre quand le courant passe, ceci est appelé commutation à chaud. Le commutateur doit dissiper de la puissance pendant la commutation à chaud. L'énergie que le commutateur doit dissiper est le produit du courant passant par le commutateur et de la tension appliquée à ce commutateur, intégré pendant la durée de transition. Si on utilise une commutation à chaud, il est nécessaire de limiter la durée de transition à quelques dizaines de microsecondes avec des commutateurs pratiques pour générateurs de chocs implantables. Par ailleurs, la durée de transition ne doit pas être trop courte, car de grandes vitesses de variation de courant ou de tension peuvent provoquer un couplage inductif ou capacitif avec des points sensibles situés ailleurs dans le circuit.

Comme il existe deux commutateurs en série pour chaque phase du choc dans la configuration à pont en H montrée à la figure 1, un seul a besoin d'être commuté à chaud. La présente invention réalise la commutation à chaud sur le côté inférieur, où il est facile de contrôler les durées de fermeture et d'ouverture.

Pour cette raison, pour fournir une phase de choc, les circuits de commande 2 commencent par appliquer HF en 11, puis attendent pendant quelques centaines de microsecondes pour donner le temps à la commande 7 du côté supérieur de fermer le commutateur 14, avant d'appliquer LF en 12. Ainsi, le commutateur 13 est déjà fermé quand le commutateur 14 commence à se fermer. Il n'y a pas de commutation à chaud pour le commutateur 13. Ainsi, la vitesse de fermeture du commutateur 14 détermine le front de montée du choc de défibrillation. La commande 10 du côté inférieur est construite comme décrit dans l'explication de la figure 1 ci-dessus pour déterminer un front de montée de choc contrôlé.

A la fin de la première phase, les circuits de commande peuvent d'abord ordonner à la commande 10 du côté inférieur d'ouvrir le commutateur 14 comme montré à la figure 14. Ou bien, si la durée d'ouverture du commutateur 13 produite par le circuit de coupure de la commande 7 du côté supérieur est adéquate, les circuits de commande peuvent ordonner simultanément à la commande du côté inférieur et à la commande du côté supérieur d'ouvrir leurs commutateurs, comme montré à la figure 13. Les circuits de commande produisent la seconde phase de façon similaire.

Une modification envisageable qui est comprise dans le champ d'application de l'invention consisterait à configurer un étage de sortie flottant comme dans les défibrillateurs implantables antérieurs. Dans ce cas, les commandes optiques selon l'invention pourraient être utilisées pour commander les commutateurs du côté inférieur comme ceux du côté supérieur, à condition qu'une commande rapide soit utilisée pour au moins un commutateur en série dans chaque phase de choc, afin de limiter les durées de transition comme expliqué ci-dessus. Un unique émetteur pourrait commander de multiples récepteurs pour faire fonctionner simultanément des commutateurs multiples pour chaque phase dans un circuit de décharge multiphasique.

Une autre modification possible comprise dans le champ d'application de l'invention consisterait à réaliser le circuit de décharge au moyen de MOSFET à canal P, d'IGBT de type P ou de transistors bipolaires pnp, à haute tension, en inversant la version montrée dans le meilleur mode de réalisation ci-dessus.

## Revendications

1. Défibrillateur implantable à générateur de chocs comprenant:
- une pile (1) pour fournir l'énergie,
- des circuits de commande (2) pour déterminer les instants de choc,
- un circuit de charge de choc (3) pour convertir l'énergie de la pile (1) en énergie de choc,
- un condensateur (4) pour stocker l'énergie de choc,
- des moyens de commutation électroniques (13, 14, 19, 20) pour relier ledit condensateur (4) à une charge (5), et
- des moyens (7, 8, 9, 10) comportant une isolation optique, pour commander lesdits moyens de commutation électroniques (13, 14, 19, 20) et comprenant :
. des moyens d'émission optiques (22) pour convertir au moins un signal électrique (27) provenant desdits circuits de commande (2) en au moins un signal optique,
. des moyens de réception optiques (23) pour convertir au moins un signal optique en au moins un signal électrique (30) pour fermer et ouvrir selectivement ledit commutateur électronique (13, 14, 19, 20), et
. un trajet optique (24A, 24B) isolé électriquement acheminant chaque signal optique provenant desdits moyens d'émission optiques (22) vers lesdits moyens de réception optiques (23),
- lesdits moyens d'émission optiques (22) émettant un signal optique dont la présence indique auxdits moyens de réception (23) de fermer ledit commutateur électronique (13, 14, 19, 20), et dont l'absence indique auxdits moyens de réception (23) d'ouvrir ledit commutateur électronique (13, 14, 19, 20),
- lesdits moyens de réception (23) comprenant:
. des moyens d'alimentation (40) pour fournir la puissance qui charge les bornes de commande dudit commutateur électronique,
. un premier commutateur à phototransistor (41) pour diriger sélectivement le courant provenant desdits moyens d'alimentation (40) vers les bornes de commande dudit commutateur électronique, pour charger les bornes de commande dudit commutateur électronique et ainsi fermer ledit commutateur électronique en réponse à la présence dudit signal optique,
**caractérisé en ce que** lesdits moyens de réception (23) comprennent :
- des moyens de coupure (42) pour décharger sélectivement les bornes de commande dudit commutateur électronique et ainsi ouvrir ledit commutateur électronique en réponse à l'absence dudit courant de charge.

2. Défibrillateur selon la revendication 1, **caractérisé en ce que** lesdits moyens d'alimentation (40) comprennent:
- un condensateur (43) pour stocker de l'énergie à basse tension,
- un circuit à courant limité, comprenant au moins une résistance (44, 45) de valeur élevée, pour charger ledit condensateur (43) pour stocker l'énergie à basse tension en provenance dudit condensateur (4) pour stocker l'énergie de choc, et
- des moyens limiteurs de tension (46) pour empêcher ledit condensateur (43) de stockage d'énergie à basse tension de se charger au-delà d'une tension préétablie.

3. Défibrillateur selon la revendication 2, **caractérisé en ce que** lesdits moyens limiteurs de tension (46) comprennent une diode de Zener avec une tension de Zener d'approximativement 15 V.

4. Défibrillateur selon l'ensemble des revendications 1 et 3, **caractérisé en ce que** lesdits moyens de coupure (42) comprennent des moyens à transistor (55) pour décharger les bornes de commande dudit commutateur électronique quand lesdits moyens d'alimentation (40) et lesdits moyens à phototransistor (41) ne fournissent plus de courant.

5. Défibrillateur selon la revendication 1, **caractérisé en ce que** lesdits moyens de réception optiques (23) comprennent:
- au moins une photodiode (48, 49) pour fournir sélectivement le courant aux bornes de commande dudit commutateur électronique pour charger les bornes de commande dudit commutateur électronique et fermer ainsi ledit commutateur électronique en réponse à la présence dudit signal optique, et
- des moyens de coupure (42) pour décharger sélectivement les bornes de commande dudit commutateur électronique et ainsi ouvrir ledit commutateur électronique, en réponse à l'absence dudit courant de charge en provenance de ladite ou desdites photodiodes.

6. Défibrillateur selon la revendication 5, **caractérisé en ce que** lesdits moyens de coupure (42) comprennent des moyens à transistor (55) pour décharger les bornes de commande dudit commutateur électronique quand ladite ou lesdites photodiodes (48, 49) ne fournissent plus de courant aux bornes de commande dudit commutateur électronique.

7. Défibrillateur selon la revendication 1, **caractérisé en ce que** lesdits moyens d'émission optiques (22) émettent un premier signal optique dont la présence indique auxdits moyens de réception (23) de fermer ledit commutateur électronique, et émettent un second signal optique dont la présence indique auxdits moyens de réception (23) d'ouvrir ledit commutateur électronique.

8. Défibrillateur selon la revendication 7, **caractérisé en ce que** lesdits moyens de réception (23) comprennent:
- des moyens d'alimentation (40) pour fournir la puissance qui charge les bornes de commande dudit commutateur électronique,
- un premier commutateur à phototransistor (41) pour faire passer sélectivement la puissance depuis lesdits moyens d'alimentation vers les bornes de commande dudit commutateur électronique, pour charger les bornes de commande dudit commutateur électronique et ainsi fermer ledit commutateur électronique, en réponse à la présence dudit premier signal optique, et
- des moyens de coupure (42) pour décharger sélectivement les bornes de commande dudit commutateur électronique et ainsi ouvrir ledit commutateur électronique en réponse à la présence dudit second signal optique.

9. Défibrillateur selon la revendication 8, **caractérisé en ce que** lesdits moyens d'alimentation (40) comprennent:
- un condensateur (43) pour stocker de l'énergie à basse tension,
- un trajet à courant limité, comprenant au moins une résistance de valeur élevée (44, 45), pour charger ledit condensateur (43) pour stocker l'énergie à basse tension à partir dudit condensateur (4) pour stocker l'énergie de choc, et
- des moyens limiteurs de tension (46) pour empêcher ledit condensateur de stockage d'énergie à basse tension (43) de se charger au-delà d'une tension préétablie.

10. Défibrillateur selon la revendication 9, **caractérisé en ce que** lesdits moyens limiteurs de tension (46) comprennent une diode de Zener avec une tension de Zener d'approximativement 15 V.

11. Défibrillateur selon l'une quelconque des revendications 8 à 10, **caractérisé en ce que** lesdits moyens dé circuit de coupure (42) comprennent:
- un second phototransistor (50) activé par ledit second signal optique, relié de façon à décharger les bornes de commande dudit commutateur électronique quand il est activé, et
- une résistance (52) pour empêcher l'accumulation de la charge auxdites bornes de commande quand aucun signal optique n'est présent.

12. Défibrillateur selon la revendication 7 **caractérisé en ce que** lesdits moyens de réception optiques (23) comprennent:
- au moins une photodiode (48, 49) pour fournir sélectivement le courant aux bornes de commande dudit commutateur électronique pour charger les bornes de commande dudit commutateur électronique et ainsi fermer ledit commutateur électronique, en réponse à la présence dudit premier signal optique, et
- des moyens de coupure (42) pour décharger sélectivement les bornes de commande dudit commutateur électronique et ainsi ouvrir ledit commutateur électronique, en réponse à la présence dudit second signal optique.

13. Défibrillateur selon la revendication 12 **caractérisé en ce que** lesdits moyens de coupure comprennent:
- un second phototransistor (50) activé par ledit second signal optique, relié de façon à décharger les bornes de commande dudit commutateur électronique quand il est activé, et
- une résistance (52) pour empêcher l'accumulation de la charge sur lesdites bornes de commande quand aucun signal optique n'est présent.

14. Défibrillateur selon l'une des revendications 1 à 13 **caractérisé en ce que** lesdits moyens de commutation électroniques comprennent deux commutateurs électroniques sur le côté supérieur (13, 19) et deux commutateurs électroniques sur le côté inférieur (14, 20), en configuration de pont en H pour relier ledit condensateur (4) à une charge (5) avec une polarité sélective .

15. Défibrillateur selon la revendication 14 **caractérisé en ce qu'**il comprend en outre une liaison (21) entre le côté inférieur dudit condensateur (4) et dudit pont en H et la masse.

16. Défibrillateur selon la revendication 14 **caractérisé en ce qu'**il comprend en outre une liaison (21) entre le côté inférieur dudit condensateur (4) et dudit pont en H et une tension d'alimentation plus négative que la masse.

17. Défibrillateur selon la revendication 16, **caractérisé en ce que** ladite tension d'alimentation est comprise entre -5 et -20 V.

18. Défibrillateur selon la revendication 16, **caractérisé en ce que** ladite tension d'alimentation est d'approximativement -15 V.

19. Défibrillateur selon l'une des revendications 15 à 18, **caractérisé en ce que** lesdits moyens (7, 8, 9, 10) pour commander les moyens de commutation électroniques (13, 14, 19, 20) comprennent:
- deux commandes isolées (7, 8) du côté supérieur pour commander sélectivement chaque commutateur (13, 19) du côté supérieur en réponse à un signal correspondant provenant desdits circuits de commande (2), et
- deux commandes (9, 10) du côté inférieur pour commander sélectivement chaque moyen de commutation (14, 20) du côté inférieur en réponse à un signal correspondant provenant desdits circuits de commande (2),
les commandes (7, 8) du côté supérieur étant isolées optiquement.

20. Défibrillateur selon la revendication 19, **caractérisé en ce que** lesdits circuits de commande (2) pour déterminer les durées de chocs activent d'abord l'une desdites commandes (7, 8) du côté supérieur, puis après une certaine durée préétablie, activent la commande correspondante (9, 10) du côté inférieur, pour commencer chaque phase d'un choc.

21. Défibrillateur selon l'une des revendications 19 ou 20, **caractérisé en ce que** lesdits circuits de commande (2) pour déterminer les durées de chocs commencent par désactiver l'une desdites commandes (7, 8) du côté supérieur, puis après une certaine durée préétablie, désactivent la commande correspondante (9, 10) du côté inférieur, pour mettre fin à chaque phase d'un choc.

22. Défibrillateur selon l'une des revendications 19 ou 20, **caractérisé en ce que** lesdits circuits de commande (2) pour déterminer les durées de chocs désactivent simultanément l'une desdites commandes (7, 8) du côté supérieur et la commande correspondante (9, 10) du côté inférieur pour mettre fin à chaque phase d'un choc.

23. Défibrillateur selon l'une des revendications 20 ou 21, **caractérisé en ce que** la durée préétablie est de l'ordre de quelques centaines de microsecondes.

## Claims

1. An implantable defibrillator with a shock generator comprising:
- a battery (1) for supplying the energy,
- control circuits (2) for determining the shock times,
- a shock charging circuit (3) for converting the energy of the battery (1) into shock energy,
- a capacitor (4) for storing the shock energy,
- electronic switching means (13, 14, 19, 20) for connecting said capacitor (4) to a load (5), and
- means (7, 8, 9, 10) comprising optical isolation, for controlling said electronic switching means (13, 14, 19, 20) and comprising:
• optical emission means (22) for converting at least one electric signal (27) coming from said control circuits (2) into at least one optical signal,
• optical reception means (23) for converting at least one optical signal into at least one electric signal (30) for selectively closing and opening said electronic switch (13, 14, 19, 20), and
• an electrically isolated optical path (24A, 24B) bringing each optical signal coming from said optical emission means (22) to said optical reception means (23),
- said optical emission means (22) emitting an optical signal the presence of which prompts said reception means (23) to close said electronic switch (13, 14, 19, 20), and the absence of which prompts said reception means (23) to open said electronic switch (13, 14, 19, 20),
- said reception means (23) comprising:
• supply means (40) for providing the power which charges the control terminals of said electronic switch,
• a first phototransistor switch (41) for selectively directing the current coming from said supply means (40) to the control terminals of said electronic switch, for charging the control terminals of said electronic switch and thus closing said electronic switch in response to the presence of said optical signal,
**characterised in that** said reception means (23) comprise:
- cutoff means (42) for selectively discharging the control terminals of said electronic switch and thus opening said electronic switch in response to the absence of said charging current.

2. A defibrillator according to Claim 1, **characterised in that** said supply means (40) comprise:
- a capacitor (43) for storing low-voltage energy,
- a limited-current circuit, comprising at least one resistor (44, 45) of high value, for charging said capacitor (43) for storing the low-voltage energy coming from said capacitor (4) for storing the shock energy, and
- voltage-limiting means (46) for preventing said capacitor (43) for storing low-voltage energy from being charged beyond a preestablished voltage.

3. A defibrillator according to Claim 2, **characterised in that** said voltage-limiting means (46) comprise a Zener diode with a Zener voltage of approximately 15 V.

4. A defibrillator according to both of Claims 1 and 3, **characterised in that** said cutoff means (42) comprise transistor means (55) for discharging the control terminals of said electronic switch when said supply means (40) and said phototransistor means (41) no longer supply current.

5. A defibrillator according to Claim 1, **characterised in that** said optical reception means (23) comprise:
- at least one photodiode (48, 49) for selectively supplying the current to the control terminals of said electronic switch, for charging the control terminals of said electronic switch and thus closing said electronic switch in response to the presence of said optical signal, and
- cutoff means (42) for selectively discharging the control terminals of said electronic switch and thus opening said electronic switch in response to the absence of said charging current coming from said photodiode(s).

6. A defibrillator according to Claim 5, **characterised in that** said cutoff means (42) comprise transistor means (55) for discharging the control terminals of said electronic switch when said photodiode(s) (48, 49) no longer supply current to the control terminals of said electronic switch.

7. A defibrillator according to Claim 1, **characterised in that** said optical emission means (22) emit a first optical signal, the presence of which prompts said reception means (23) to close said electronic switch, and emit a second optical signal, the presence of which prompts said reception means (23) to open said electronic switch.

8. A defibrillator according to Claim 7, **characterised in that** said reception means (23) comprise:
- supply means (40) for providing the power which charges the control terminals of said electronic switch,
- a first phototransistor switch (41) for causing the power to pass selectively from said supply means to the control terminals of said electronic switch, for charging the control terminals of said electronic switch and thus closing said electronic switch, in response to the presence of said first optical signal, and
- cutoff means (42) for selectively discharging the control terminals of said electronic switch and thus opening said electronic switch in response to the presence of said second optical signal.

9. A defibrillator according to Claim 8, **characterised in that** said supply means (40) comprise:
- a capacitor (43) for storing low-voltage energy,
- a limited-current path, comprising at least one resistor (44, 45) of high value, for charging said capacitor (43) for storing the low-voltage energy coming from said capacitor (4) for storing the shock energy, and
- voltage-limiting means (46) for preventing said capacitor (43) for storing low-voltage energy from being charged beyond a preestablished voltage.

10. A defibrillator according to Claim 9, **characterised in that** said voltage-limiting means (46) comprise a Zener diode with a Zener voltage of approximately 15 V.

11. A defibrillator according to any one of Claims 8 to 10, **characterised in that** said cutoff circuit means (42) comprise:
- a second phototransistor (50) activated by said second optical signal, connected so as to discharge the control terminals of said electronic switch when it is activated, and
- a resistor (52) for preventing the accumulation of the charge at said control terminals when no optical signal is present.

12. A defibrillator according to Claim 7, **characterised in that** said optical reception means (23) comprise:
- at least one photodiode (48, 49) for selectively supplying the current to the control terminals of said electronic switch, for charging the control terminals of said electronic switch and thus closing said electronic switch in response to the presence of said first optical signal, and
- cutoff means (42) for selectively discharging the control terminals of said electronic switch and thus opening said electronic switch in response to the presence of said second optical signal.

13. A defibrillator according to Claim 12, **characterised in that** said cutoff means comprise:
- a second phototransistor (50) activated by said second optical signal, connected so as to discharge the control terminals of said electronic switch when it is activated, and
- a resistor (52) for preventing the accumulation of the charge on said control terminals when no optical signal is present.

14. A defibrillator according to one of Claims 1 to 13, **characterised in that** said electronic switching means comprise two electronic switches on the upper side (13, 19) and two electronic switches on the lower side (14, 20), in an H-shaped bridge configuration for connecting said capacitor (4) to a load (5) with a selective polarity.

15. A defibrillator according to Claim 14, **characterised in that** it furthermore comprises a connection (21) between the lower side of said capacitor (4) and said H-shaped bridge and the earth.

16. A defibrillator according to Claim 14, **characterised in that** it furthermore comprises a connection (21) between the lower side of said capacitor (4) and said H-shaped bridge and a supply voltage which is more negative than the earth.

17. A defibrillator according to Claim 16, **characterised in that** said supply voltage is of between -5 and -20 V.

18. A defibrillator according to Claim 16, **characterised in that** said supply voltage is of approximately -15 V.

19. A defibrillator according to one of Claims 15 to 18, **characterised in that** said means (7, 8, 9, 10) for controlling the electronic switching means (13, 14, 19, 20) comprise:
- two isolated controls (7, 8) on the upper side for selectively controlling each switch (13, 19) on the upper side in response to a corresponding signal coming from said control circuits (2), and
- two controls (9, 10) on the lower side for selectively controlling each switching means (14, 20) on the lower side in response to a corresponding signal coming from said control circuits (2),
the controls (7, 8) of the upper side being isolated optically.

20. A defibrillator according to Claim 19, **characterised in that** said control circuits (2) for determining the shock durations first of all activate one of said controls (7, 8) on the upper side, then, after a certain pre-established delay, activate the corresponding control (9, 10) on the lower side, for starting each phase of a shock.

21. A defibrillator according to one of Claims 19 or 20, **characterised in that** said control circuits (2) for determining the durations of shocks start by deactivating one of said controls (7, 8) on the upper side, then after a certain pre-established duration, deactivate the corresponding control (9, 10) on the lower side, for ending each phase of a shock.

22. A defibrillator according to one of Claims 19 or 20, **characterised in that** said control circuits (2) for determining the durations of shocks simultaneously deactivate one of said controls (7, 8) on the upper side and the corresponding control (9, 10) on the lower side, for ending each phase of a shock.

23. A defibrillator according to one of Claims 20 or 21, **characterised in that** the pre-established duration is of the order of several hundreds of microseconds.

## Patentansprüche

1. Implantierbarer Defibrillator mit einem Elektroschockgenerator, der aufweist:
- eine Batterie (1) zur Energiezufuhr,
- Steuerschaltungen (2) zum Bestimmen der Schockzeitpunkte,
- eine Schockladeschaltung (3) zum Umwandeln der Energie der Batterie (1) in Schockenergie,
- einen Kondensator (4) zum Speichern der Schockenergie,
- elektronische Schaltungseinrichtungen (13, 14, 19, 20) zum Verbinden des Kondensators (4) mit einer Last (5) und
- Einrichtungen (7, 8, 9, 10) mit einer optischen Isolation zum Steuern der elektronischen Schaltungseinrichtungen (13, 14, 19, 20), die aufweisen:
optische Sendeeinrichtungen (22) zum Umwandeln mindestens eines, von den Steuerschaltungen (2) ausgehenden elektrischen Signals (27) in mindestens ein optisches Signal,
optische Empfangseinrichtungen (23) zum Umwandeln mindestens eines optischen Signals in mindestens ein elektrisches Signal (30), um den elektronischen Schalter (13, 14, 19, 20) selektiv zu öffnen und zu schließen, und
einen elektrisch isolierten Lichtweg (24A, 24B), der jedes von den optischen Sendeeinrichtungen (22) ausgehende optische Signal zu den optischen Empfangseinrichtungen (23) leitet,
wobei
- die optischen Sendeeinrichtungen (22) ein optisches Signal aussenden, dessen Vorhandensein die Empfangseinrichtungen (23) darauf hinweist, den elektronischen Schalter (13, 14, 19, 20) zu schließen, und dessen Fehlen die Empfangseinrichtungen (23) darauf hinweist, den elektronischen Schalter (13, 14, 19, 20) zu öffnen, wobei
- die Empfangseinrichtungen (23) aufweisen:
· Versorgungseinrichtungen (40) zum Zuführen der Leistung, die die Steuerklemmen des elektronischen Schalters auflädt,
· einen ersten Phototransistor-Schalter (41) zum selektiven Lenken des von den Versorgungseinrichtungen (40) ausgehenden Stroms zu den Steuerklemmen des elektronischen Schalters, um die Steuerklemmen des elektronischen Schalters aufzuladen und somit den elektronischen Schalter im Ansprechen auf das Vorhandensein des optischen Signals zu schließen,
**dadurch gekennzeichnet, dass** die Empfangseinrichtungen (23) aufweisen:
- Abschaltungseinrichtungen (42), um die Steuerklemmen des elektronischen Schalters selektiv zu entladen und somit den elektronischen Schalter im Ansprechen auf das Fehlen des Ladestroms zu öffnen.

2. Defibrillator nach Anspruch 1, **dadurch gekennzeichnet, dass** die Versorgungseinrichtungen (40) aufweisen:
- einen Kondensator (43) zum Speichern von Niedrigspannungsenergie,
- eine Strombegrenzerschaltung mit mindestens einem hochohmigen Widerstand (44, 45) zum Aufladen des Kondensators (43) zum Speichern der vom Kondensator (4) ausgehenden Niedrigspannungsenergie zum Speichern der Schockenergie, und
- Spannungsbegrenzungseinrichtungen (46), um zu verhindern, dass der Kondensator (43) zum Speichern von Niedrigspannungsenergie sich über eine festgelegte Spannung hinaus auflädt.

3. Defibrillator nach Anspruch 2, **dadurch gekennzeichnet, dass** die Spannungsbegrenzungseinrichtungen (46) eine Zener-Diode mit einer Zener-Spannung von ungefähr 15 V auf weisen.

4. Defibrillator nach der Gesamtheit der Ansprüche 1 und 3, **dadurch gekennzeichnet, dass** die Abschalteinrichtungen (42) Transistoreinrichtungen (55) aufweisen, um die Steuerklemmen des elektronischen Schalters zu entladen, wenn die Versorgungseinrichtungen (40) und die Phototransistoreinrichtungen (41) keinen Strom mehr zuführen.

5. Defibrillator nach Anspruch 1, **dadurch gekennzeichnet, dass** die optischen Empfangseinrichtungen (23) aufweisen:
- mindestens eine Photodiode (48, 49) zum selektiven Zuführen von Strom zu den Steuerklemmen des elektronischen Schalters, um die Steuerklemmen des elektronischen Schalters aufzuladen und somit den elektronischen Schalter im Ansprechen auf das Vorhandensein des optischen Signals zu schließen, und
- Abschalteinrichtungen (42), um die Steuerklemmen des elektronischen Schalters selektiv zu entladen und somit den elektronischen Schalter im Ansprechen auf das Fehlen des Ladungsstroms von der Photodiode oder den Photodioden zu öffnen.

6. Defibrillator nach Anspruch 5, **dadurch gekennzeichnet, dass** die Abschalteinrichtungen (42) Transistoreinrichtungen (55) zum Entladen der Steuerklemmen des elektronischen Schalters aufweisen, wenn die Photodiode oder die Photodioden (48, 49) den Steuerklemmen des elektronischen Schalters keinen Strom mehr zuführen.

7. Defibrillator nach Anspruch 1, **dadurch gekennzeichnet, dass** die optischen Sendeeinrichtungen (22) ein erstes optisches Signal aussenden, dessen Vorhandensein die Empfangseinrichtungen (23) darauf hinweist, den elektronischen Schalter zu schließen, und die ein zweites optisches Signal aussenden, dessen Vorhandensein die Empfangseinrichtungen (23) darauf hinweist, den elektronischen Schalter zu öffnen.

8. Defibrillator nach Anspruch 7, **dadurch gekennzeichnet, dass** die Empfangseinrichtungen aufweisen:
- Versorgungseinrichtungen (40) zum Zuführen der die Steuerklemmen des elektronischen Schalters aufladenden Leistung,
- einen ersten Phototransistor-Schalter (41) zum selektiven Leiten der Leistung von den Versorgungseinrichtungen zu den Steuerklemmen des elektronischen Schalters, zum Laden der Steuerklemmen des elektronischen Schalters und somit zum Schließen des elektronischen Schalters im Ansprechen auf das Vorhandensein des ersten optischen Signals und
- Abschalteinrichtungen (42) zum selektiven Entladen der Steuerklemmen des elektronischen Schalters und somit zum Öffnen des elektronischen Schalters im Ansprechen auf das Vorhandensein des zweiten optischen Signals.

9. Defibrillator nach Anspruch 8, **dadurch gekennzeichnet, dass** die Versorgungseinrichtungen (40) aufweisen:
- einen Kondensator (43) zum Speichern der Niedrigspannungsenergie,
- einen Strombegrenzerweg mit mindestens einem hochohmigen Widerstand (44, 45) zum Aufladen des Kondensators (43) zum Speichern der von dem Kondensator (4) ausgehenden Niedrigspannungsenergie zum Speichern der Schockenergie und
- Spannungsbegrenzungseinrichtungen (46), um zu verhindern, dass sich der Kondensator zum Speichern von Niedrigspannungsenergie (43) über eine festgelegte Spannung hinaus auflädt.

10. Defibrillator nach Anspruch 9, **dadurch gekennzeichnet, dass** die Spannungsbegrenzungs-Einrichtungen (46) eine Zener-Diode mit einer Zener-Spannung von ungefähr 15 V umfassen.

11. Defibrillator nach einem der Ansprüche 8 bis 10, **dadurch gekennzeichnet, dass** die Abschalteinrichtungen (42) aufweisen:
- einen zweiten Phototransistor (50), der durch das zweite optische Signal aktiviert wird und so angeschlossen ist, dass er die Steuerklemmen des elektronischen Schalters entlädt, wenn er aktiviert ist, und
- einen Widerstand (52), um zu verhindern, dass sich Ladung an den Steuerklemmen akkumuliert, wenn kein optisches Signal vorhanden ist.

12. Defibrillator nach Anspruch 7, **dadurch gekennzeichnet, dass** die optischen Empfangseinrichtungen (23) aufweisen:
- mindestens eine Photodiode (48, 49) zum selektiven Liefern von Strom zu den Steuerklemmen des elektronischen Schalters zum Laden der Steuerklemmen des elektronischen Schalters und somit zum Schließen des elektronischen Schalters im Ansprechen auf das Vorhandensein des ersten optischen Signals und
- Abschaltungseinrichtungen (42) zum selektiven Entladen der Steuerklemmen des elektronischen Schalters und somit zum Öffnen des elektronischen Schalters im Ansprechen auf das Vorhandensein des zweiten optischen Signals.

13. Defibrillator nach Anspruch 12, **dadurch gekennzeichnet, dass** die Abschaltungseinrichtungen aufweisen:
- einen zweiten Phototransistor (50), der durch das zweite optische Signal aktiviert wird und derart angeschlossen ist, dass er die Steuerklemmen des elektronischen Schalters entlädt, wenn er aktiviert ist, und
- einen Widerstand (52), um zu verhindern, dass sich Ladung an den Steuerklemmen akkumuliert, wenn kein optisches Signal vorhanden ist.

14. Defibrillator nach einem der Ansprüche 1 bis 13, **dadurch gekennzeichnet, dass** die elektronischen Schaltungseinrichtungen zwei elektronische Schalter auf der Oberseite (13, 19) und zwei elektronische Schalter auf der Unterseite (14, 20) bei einer H-Brückenschaltung aufweisen, um den Kondensator (4) an eine Last (5) mit einer selektiven Polarität anzuschließen.

15. Defibrillator nach Anspruch 14, **dadurch gekennzeichnet, dass** er des Weiteren eine Verbindung zwischen der Unterseite des Kondensators (4) und der H-Brücke und Masse aufweist.

16. Defibrillator nach Anspruch 14, **dadurch gekennzeichnet, dass** er des Weiteren eine Verbindung (21) zwischen der Unterseite des Kondensators (4) und der H-Brücke und einer Versorgungsspannung aufweist, die negativer als Masse ist.

17. Defibrillator nach Anspruch 16, **dadurch gekennzeichnet, dass** die Versorgungsspannung zwischen -5 und -20V liegt.

18. Defibrillator nach Anspruch 16, **dadurch gekennzeichnet, dass** die Versorgungsspannung ungefähr -15 V beträgt.

19. Defibrillator nach einem der Ansprüche 15 bis 18, **dadurch gekennzeichnet, dass** die Einrichtungen (7, 8, 9, 10) zum Steuern der elektronischen Schaltungseinrichtungen (13, 14, 19, 20) aufweisen:
- zwei isolierte Steuerungen (7, 8) an der Oberseite zum selektiven Steuern jedes Schalters. (13, 19) der Oberseite im Ansprechen auf ein von den Steuerschaltungen (2) ausgehendes, entsprechendes Signal und
- zwei Steuerungen (9, 10) an der Unterseite zum selektiven Steuern jeder Schaltungseinrichtung (14, 20) der Unterseite im Ansprechen auf ein von den Steuerschaltungen (2) ausgehendes, entsprechendes Signal,
wobei die Steuerungen (7, 8) an der Oberseite optisch isoliert sind.

20. Defibrillator nach Anspruch 19, **dadurch gekennzeichnet, dass** die Steuerschaltungen (2) zum Bestimmen der Schockzeitdauern zuerst eine der Steuerungen (7, 8) an der Oberseite aktivieren, dann, nach einer bestimmten, vorher festgelegten Zeitdauer, die entsprechende Steuerung (9, 10) an der Unterseite aktivieren, um jede Phase eines Schocks einzuleiten.

21. Defibrillator nach einem der Ansprüche 19 oder 20, **dadurch gekennzeichnet, dass** die Steuerschaltungen (2) zum Bestimmen der Schockzeitdauern damit beginnen, eine der Steuerungen (7, 8) an der Oberseite zu deaktivieren, dann, nach einer bestimmten, vorher festgelegten Zeitdauer die entsprechende Steuerung (9, 10) an der Unterseite deaktivieren, um jede Phase eines Schocks zu beenden.

22. Defibrillator nach einem der Ansprüche 19 oder 20, **dadurch gekennzeichnet, dass** die Steuerschaltungen (2) zum Bestimmen der Schockzeitdauern simultan eine der Steuerungen (7, 8) an der Oberseite und die entsprechende Steuerung (9, 10) an der Unterseite deaktivieren, um jede Phase eines Schocks zu beenden.

23. Defibrillator nach einem der Ansprüche 20 oder 21, **dadurch gekennzeichnet, dass** die vorher festgelegte Zeitdauer im Bereich von einigen Hundert Mikrosekunden liegt.
